# EUROPEAN PATENT SPECIFICATION

(11) **EP 3 796 858 B1**
(45) Date of publication and mention of the grant of the patent: **20.08.2025**
(21) Application number: 19726298.3
(22) Date of filing: 14.05.2019
(51) Int. Cl.: A61B 18/14

(54) **LUMEN REINFORCEMENT DEVICE AND SYSTEM**
LUMENVERSTÄRKUNGSVORRICHTUNG UND SYSTEM
DISPOSITIF ET SYSTÈME DE RENFORCEMENT DE LUMIÈRE

(30) Priority: 21.05.2018 US 201862674207 P
(43) Date of publication of application: 31.03.2021
(73) Proprietor: Smith&Nephew, Inc., Memphis, Tennessee 38116 (US); Smith & Nephew Orthopaedics AG, 6300 Zug (CH)
(72) Inventor: EVANS, Douglas G., Austin, Texas 78749 (US); TURNER, David, Austin, Texas 78735 (US); SMITH, Brendan, Austin, Texas 78734 (US)
(74) Representative: White, Andrew John
(86) International application number: PCT/US2019/032240
(87) International publication number: WO 2019/226419

(56) References cited:
- US-A- 5 972 012
- US-A1- 2007 106 292
- US-A1- 2016 339 207

## Description

### CROSS-REFERENCE TO RELATED APPLICATIONS

This application claims the benefit of U.S Provisional Application No. 62/674,207 filed May 21, 2018 entitled "LUMEN REINFORCEMENT DEVICE AND SYSTEM".

### FIELD OF THE INVENTION

The present invention relates generally to the field of surgical instruments, and more particularly relates to surgical instruments providing a passageway (lumen) through which fluids or other instrument components may be passed that is reinforced to prevent passageway collapse under bending forces. The instrument of some embodiments is a component of an electrosurgical wand in an electrosurgical system.

### BACKGROUND

Surgical instruments with passageways, such as a shaft and internal lumen of an electrosurgical device, need to be bent during the performance of some surgical procedures, including but not limited to adenoidectomy. Such devices typically incorporate a suction or supply lumen for fluid and tissue passage through the shaft, which is being bent. Because the lumen may be made of a polymer and be operated under negative pressure and elevated temperature, the lumen may be prone to kinking when bent to a tight radius or bend angle. Similarly, the outer shaft around the lumen may be made from a metal that is a relatively soft annealed metal shaft. This metal shaft may be prone to kinking when bent to typical operating conditions. Such bending and kinking may restrict or even stop flow through the lumen.

Some manufacturers address flow restriction through lumen restriction by publishing bending guidelines in product Instructions For Use, recommending maximum bend angles to users. A reusable handheld bending guide to encourage a safe bend radius may also be provided. This solution may not allow a user to bend the device as much as desired for unique patient anatomies, and the bending guide is subject to being misplaced. Another prior art approach is to use more rigid, and expensive, Polyether ether ketone (PEEK) tubing and/or thicker tube walls to help increase the bending and collapse resistance of the lumen. These measures only address the problem in part. In addition to increased cost and geometric constraints within the device itself, where assembly and design clearances are often limited, these measures are unable to address the alternative failure mode in which the outer shaft kinks and crushes the lumen. It is also known in the prior art (especially with cardiovascular catheter instruments) to use flexible lumens that are reinforced by over-molding braided ribbons or very thin wires to provide structure within the tube walls. This is a relatively expensive solution that provides only limited support to the lumen in a configuration required for performing electrosurgical procedures. Such reinforced extrusions are effective at providing lumen support for torsion and axial resistance required for insertion of a catheter into tortuous cardiovascular anatomies, but ineffective at protecting a lumen from external crushing forces. Some manufacturers, such as is illustrated with Covidien's monopolar suction cautery device, have addressed the challenge by providing a polymer mandrel, such as a PTFE rod or filament, to be inserted through a shaft that serves as the suction passageway. Shaft integrity is enhanced by use of an aluminum shaft to prevent kinking. The polymer mandrel provides internal support for the lumen during bending and is removed to reveal the passageway that may be used while the device is bent. This approach incorporates an additional component and increases total cost. This approach also requires additional steps by an end user who, in addition to inserting and removing the mandrel each time a bend is desired, must also ensure that the mandrel remains readily accessible and sterile throughout the procedure.

It would be advantageous to provide surgical instruments that provide for adequate bending angles of the shaft and lumen to meet surgical needs while preventing restriction of passageways during bending. It may be further advantageous if instrument embodiments would provide enough bending resistance to prevent the instrument embodiments from being damaged when being subjected to typical use forces. It would be a cost advantage if components of the surgical instruments could be fabricated from off-the-shelf components more readily than prior art solutions that require over-molding and other relatively expensive manufacturing procedures.

US 2016/0339207 A1 describes a catheter including a distal tip electrode and a flexible shaft that comprises a multi-layered coil member and a central lumen. It also includes irrigation tubing for delivering irrigation fluid to the distal tip electrode.

US 5,972,012 describes surgical apparatus with an end region containing a mechanical cutting head and end region and means for articulating the end-region to access hard to-reach portions of the anatomy. The apparatus includes an elongated shaft that comprises an articulation region comprising helical wire.

US 2007/106292 A1 describes a device that includes a flexible sheath, an elongate shaft, and a distal portion that includes an energy transfer element such as an electrode. A coiled wire may be provided as a reinforcing element to prevent kinking or collapse of the shaft. Fluid may also be delivered through the shaft.

### SUMMARY

The invention is defined in the appended claims.

An embodiment of the disclosure is a lumen that includes at least an inner tubular member with an inside diameter and an outside diameter along its length and a support coil. Embodiments of the support coil have an inside diameter and an outside diameter along its length, the support coil comprising a helically formed wire with a wire diameter, the wire extending helically along the length of the support coil to produce walls of the support coil. The walls of the support coil may be defined by extents of the wire at the inside diameter of the support coil and the outside diameter of the support coil. Substantially all of each revolution of the wire about the length of the support coil of some embodiments occurs within a respective distance along the length of the support coil of three wire diameters.

Another embodiment of the disclosure is an electrosurgical wand with a wand body having a proximal end and a distal end, wherein the distal end includes at least a portion that is flexible, a fluid supply port coupled near the proximal end of the wand body and communicating through the wand body to facilitate the provision of fluid at or near the distal end of the wand body, a suction port coupled near the proximal end of the wand body and communicating through the wand body to facilitate the provision of negative pressure at or near the distal end of the wand body, a lumen, and an electrosurgical conductor passing through at least a portion of the wand body to supply tissue affecting energy at or near the distal end of the wand body. Embodiments of the lumen are located within at least a part of the portion of the distal end that is flexible and provide a passageway. Lumen embodiments may include an inner tubular member with an inside diameter and an outside diameter along its length and a support coil. Embodiments of the support coil have an inside diameter and an outside diameter along its length, the support coil comprising a helically formed wire with a wire diameter, the wire extending helically along the length of the support coil to produce walls of the support coil. The walls of the support coil may be defined by extents of the wire at the inside diameter of the support coil and the outside diameter of the support coil. Substantially all of each revolution of the wire about the length of the support coil of some embodiments occurs within a respective distance along the length of the support coil of three wire diameters.

Yet another embodiment of the disclosure is an electrosurgical system that includes at least a controller, a fluid control unit electrically coupled to the controller, the fluid control unit configured to provide fluids at a rate coordinated with operation of the controller, and an electrosurgical wand electrically coupled to the controller and fluidly coupled to the fluid control unit. The electrosurgical wand may include a wand body with a proximal end and a distal end, wherein the distal end includes at least a portion that is flexible, a fluid supply port coupled between the fluid control unit and near the proximal end of the wand body and communicating through the wand body to facilitate the provision of fluid at or near the distal end of the wand body, a suction port coupled near the proximal end of the wand body and communicating through the wand body to facilitate the provision of negative pressure at or near the distal end of the wand body, and a lumen.

The lumen may be located within at least a part of the portion of the distal end that is flexible, the lumen providing a passageway. The lumen may include at least an inner tubular member with an inside diameter and an outside diameter along its length, and a support coil with an inside diameter and an outside diameter along its length, the support coil comprising a helically formed wire with a wire diameter, the wire extending helically along the length of the support coil to produce walls of the support coil. The walls of the support coil may be defined by extents of the wire at the inside diameter of the support coil and the outside diameter of the support coil. Substantially all of each revolution of the wire about the length of the support coil may occur within a respective distance along the length of the support coil of three wire diameters. The electrosurgical wand may also include an electrosurgical conductor electrically coupled to the controller and passing through at least a portion of the wand body to supply tissue affecting energy at or near the distal end of the wand body.

Still another embodiment of the disclosure is a method of manufacturing a medical device that may include at least expanding a support coil with an inside diameter and an outside diameter along its length, the support coil comprising a helically formed wire with a wire diameter, the wire extending helically along the length of the support coil to produce walls of the support coil, inserting an inner tubular member with an inside diameter and an outside diameter along its length into the support coil inside diameter while the support coil is expanded, and causing the support coil to constrict around the inner tubular member such that the inside diameter of the support coil closely conforms to the outside diameter of the inner tubular member when the support coil is substantially straight along substantially all of its length.

### BRIEF DESCRIPTION OF THE DRAWINGS

FIG. 1 is a partially exploded system perspective view of an embodiment of an electrosurgical system.
FIG. 2 is a side elevation view of an electrosurgical wand of the system of FIG. 1 illustrating an exterior of the wand body.
FIG. 3 is an enlarged distal portion of the electrosurgical wand of FIG. 2.
FIG. 4 is a partial cross-sectional view of the enlarged distal portion of the electrosurgical wand of FIG. 3 where a support coil of the lumen has not also been cross-sectioned.
FIG. 5 is a cross-sectional view of the enlarged distal portion of the electrosurgical wand of FIG. 3 where the support coil of the lumen has also been cross-sectioned.
FIG. 6 is a further enlarged cross-sectional view showing only the support coil and the inner tubular member of the lumen.
FIG. 7 is a side elevation view of an embodiment of the electrosurgical wand of the system of FIG. 1 with a distal portion of the electrosurgical wand in a bent state.
FIG. 8 is a cross-sectional view of the bent distal portion of the electrosurgical wand illustrated in FIG. 7.

### DETAILED DESCRIPTION

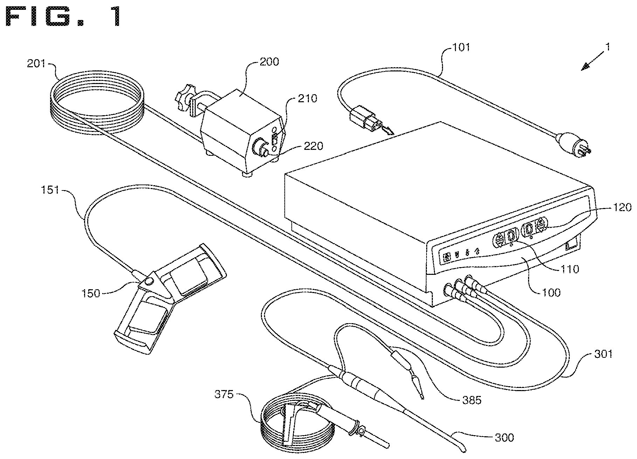

Components of an embodiment of an electrosurgical system 1 are illustrated in FIG. 1. The electrosurgical system 1 depicted includes a fluid control unit 200 electrically coupled to a controller 100 by a cable 201. An electrosurgical wand 300 is shown electrically coupled to the controller 100 by a cable 301. The controller 100 may optionally receive control signals from one or more external switching devices 150 electrically coupled through cable 151 to the controller 100. In the illustrated embodiment, the one or more switching devices 150 are foot pedals, but in other embodiments may be or include one or more of knobs, buttons, other adjustment mechanisms, and level settings. The controller 100 may be, by way of non-limiting example, a COBLATOR II controller offered for sale by Smith and Nephew. The controller 100 shown includes a tissue removal intensity setting 110 and a vasculature coagulation intensity setting 120. The controller 100 illustrated also includes electrical jacks for receiving cables 151, 201, and 301 as described above, and a power cord 101.

The fluid control unit 200 is configured to provide fluids at a rate coordinated with operation of the controller 100. For example, when tissue removal is switched on at the controller 100, or at external devices such as the external switching devices 150, fluid may be released from the fluid control unit 200. By way of non-limiting example, fluid may be a saline solution that works in conjunction with the electrosurgical wand 300 to accomplish cold ablation of tissue. The fluid control unit 200 may also include or be connected with a fluid reservoir such as an IV bag and may include a power switch 210 and a dial 220 or other control to designate a flow rate from the device.

In addition to electrical coupling to the controller 100 by the cable 301, the electrosurgical wand 300 may be fluidly coupled to the fluid control unit 200 by an irrigant tube 375. The electrosurgical wand 300 may also include a suction port 380 through which a negative pressure may be applied to the electrosurgical wand 300. A suction tube 385 may couple to the suction port 380 and be connected to an operating room suction system having, by way of non-limiting example, a suction pressure in the range of 250-350mm Hg. In alternative embodiments, suction portion may be connected to a fluid control unit configured to control a negative pressure or rate of fluid removal from the tissue treatment site.

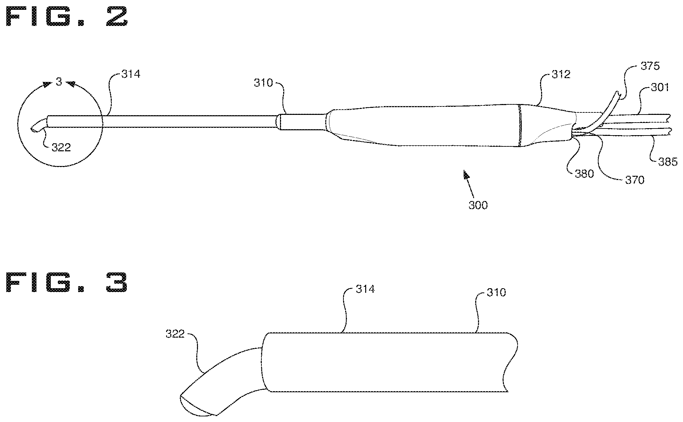

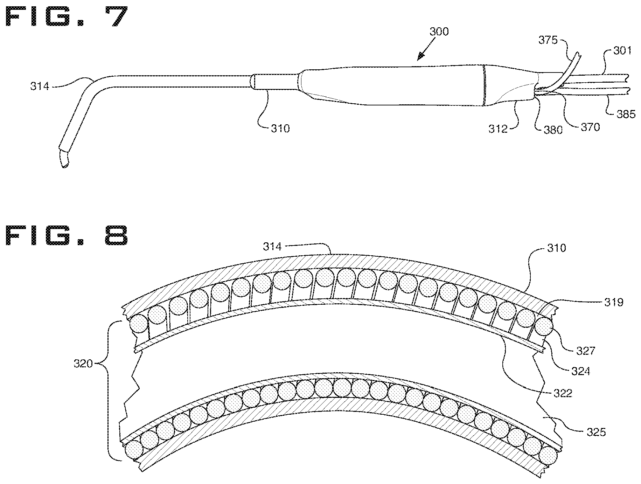

An embodiment of the electrosurgical wand 300 and its components are illustrated in more detail in FIGS. 2-8. The electrosurgical wand 300 illustrated includes a wand body 310 with a proximal end 312 and a distal end 314. The distal end 314 includes at least a portion that is flexible, as specifically illustrated in FIGS. 7 and 8. The flexible portion of the distal end 314 in some embodiments includes annealed stainless steel. More particularly, some embodiments include annealed SAE International 304 stainless steel, also known as A2 stainless steel. In some embodiments, the wand body 310 includes a 7.5 gauge hypodermic tube with a nominal outside diameter of about 4.4 mm and a wall thickness of about 0.28 mm. Other metallic or non-metallic materials such as polymers or other constructs with the ability to flex may be used in other embodiments. In some embodiments, the term "flexible" includes the ability to be deformed to a particular shape and hold that shape without continuing application of force until deformed to another shape or returned to an original shape. As used herein, a material that is more "flexible" than another material is a material that deforms more when subjected to the same load when it has the same shape as the other material.

A fluid supply port 370 coupled between the fluid control unit 200 and near the proximal end 312 of the wand body 310 and communicating through the wand body 310 to facilitate the provision of fluid at or near the distal end 314 of the wand body 310 is shown in FIGS. 2 and 7. A suction port 380 coupled near the proximal end 312 of the wand body 310 and communicating through the wand body 310 to facilitate the provision of negative pressure at or near the distal end 314 of the wand body 310 is also illustrated in FIGS. 2 and 7. In other embodiments, one or both of a fluid supply port and a suction port may be located at other locations on an electrosurgical wand. In other embodiments, only one of either a fluid supply port and or a suction port may be present.

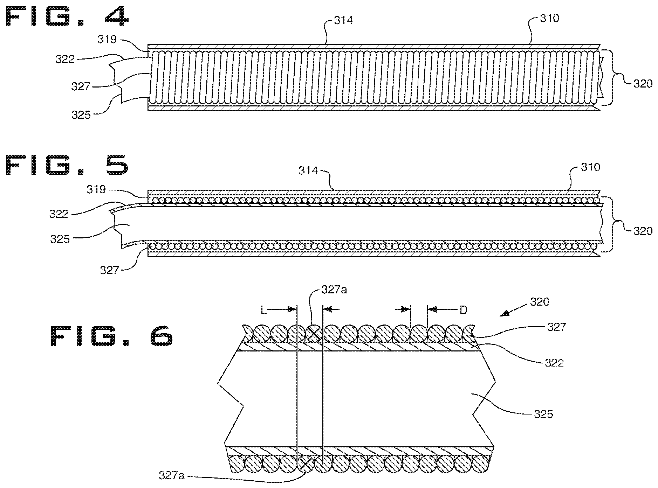

The electrosurgical wand 300 embodiment illustrated includes a lumen 320 within at least a part of the portion of the distal end 314 of the wand body 310 that is flexible, as illustrated at least in FIGS. 4, 5, 6, and 8. The term "lumen" is sometimes used to describe openings in tissue such as organ, but is used herein to refer to a tubular structure that is not necessarily an opening in tissue. The lumen 320 shown provides a passageway 325. The lumen 320 includes an inner tubular member 322 with an inside diameter and an outside diameter along its length. Embodiments of the inner tubular member 322 have solid walls along substantially all of the length of the inner tubular member 322. Other embodiments may have lateral openings into or out of an inner tubular member embodiment. In some embodiments, the wand body 310 is not only flexible, but also plastically deformable so that it will hold a particular shape when positioned to that shape. All or a portion of the distal end 314 is plastically deformable. The plastically deformable portion includes an annealed metal or other malleable material that retains a bent shape while still providing support to the lumen.

The inner tubular member 322 may in whole or in part be made from polyetheretherketone. In some embodiments, the inner tubular member 322 is substantially round and has an outside diameter of about 2.1 mm and a wall thickness of about 0.13 mm. This material and size selection enables some flexibility of the inner tubular member 322 while also providing some stiffness and some resistance to being crushed or collapsed under normal use. Additionally an inner tubular member that is electrically insulative may be preferable, to limit the current path between electrodes of the electrosurgical wand. An electrically conductive material may allow electrical currents to bridge to other unintended portions of the wand as the electrically conductive fluid travels there-along.

The electrosurgical wand 300 embodiment illustrated also includes a support coil 327 with an inside diameter and an outside diameter along its length. In some embodiments, the support coil 327 is substantially round and has an outside diameter of about 2.9 mm and a wall thickness of about 0.33 mm. The support coil 327 depicted has a helically formed metal wire with a wire diameter "D" (FIG. 6). In the illustrated embodiment, wall thickness of the support coil 327 and D are the same. The wire extends helically along the length of the support coil 327 to produce walls of the support coil 327. The walls of the support coil 327 shown are defined by extents of the wire at the inside diameter of the support coil 327 and the outside diameter of the support coil 327. In other embodiments, the wire may be a non-metallic but resilient material, for example, a relatively rigid polymer. A support element of some embodiments may be a spiral cut metallic or non-metallic tube. In some embodiments, the support element of a lumen may be a series of stacked rings not coupled to one another, but rather constrained on their ends. Stacked ring embodiments may also be coupled to one another at limited locations or coupled to an inner tubular member.

As particularly illustrated in FIG. 6, substantially all of each revolution of the wire about the length of the support coil 327 occurs within a respective distance along the length of the support coil 327 of two wire diameters "D". A distance of a revolution of the wire along the length of the support coil 327 is labelled with the designator "L", and cross-sections of a single revolution of the wire are "x" marked as elements 327a in FIG. 6. In other embodiments, substantially all of each revolution of the wire about the length of a support coil may occur within a respective distance along the length of the support coil of three wire diameters. Relatively close spacing of wires of the support coil (tight winding) may be useful to provide hoop strength as a result of the configuration of the wire and thereby provide greater crushing resistance for the lumen. In the illustrated embodiment, the wire of the support coil 327 laterally touches adjacent revolutions of the wire of the support coil 327 along substantially the entire length of the support coil 327. In some embodiments, the wire of the support coil 327 may have an axial pre-stress along the length of the wire that draws the support coil 327 into a compressed state. Such a pre-stress may be created by heat treatment, by mechanical force application, or by any other effective technique or process.

As shown in FIGS. 4-6, the inside diameter of the support coil 327 closely conforms to the outside diameter of the inner tubular member 322 when the support coil 327 is substantially straight along substantially all of its length. Substantially straight may be defined as a support coil 327 with a deviation of its longitudinal axis of less than about 15 degrees along the support coil longitudinal axis. Some embodiments include a distance between the inside diameter of the support coil 327 and the outside diameter of the inner tubular member 322 of about 0.13 mm on average. In some embodiments, the support coil 327 and the inner tubular member 322 are in contact along substantially the entire length of the support coil 327. It may also be that the support coil 327 compresses against and indents into the inner tubular member 322. In some embodiments, the support coil 327 must be radially expanded to fit around the inner tubular member 322 in the process of assembling the components. This may be accomplished by counter-rotating the support coil 327 about its longitudinal axis or by any other effective action. In still other embodiments, the outside diameter of the support coil may closely conform to the inside diameter of a wand body in which the support coil is positioned, and a space may be left between the support coil and an inner tubular member. In an alternative embodiment, an outside diameter of a support coil may closely conform to an inside diameter of a wand body in which the support coil is positioned. This configuration would limit potential crushing or collapse on an associated inner tubular member by also adding the strength of the wand body to the construct without necessarily having the support coil closely conform to the inner tubular member.

In addition to the passageway 325, a transfer space 319 may exist between the wand body 310 the lumen 320, as illustrated in FIGS. 4, 5, and 8. Either of the passageway 325 or the transfer space 319 may be used to pass fluid between more proximal and distal portions of the wand body 310. In some embodiments, the passageway 325 of the inner tubular member 322 is a suction tube that is coupled to a negative pressure source through, for example, the suction port 380. Likewise, the transfer space 319 may be open to a negative pressure source through, for example, the suction port 380 in other embodiments. The reciprocal may be true of other embodiments where the fluid supply port 370 may be in fluid communication with either of the passageway 325 of the inner tubular member 322 or the transfer space in respective embodiments.

Electrosurgical system embodiments may also include an electrosurgical conductor electrically coupled to the controller 100 and passing through at least a portion of the wand body 310 to supply tissue affecting energy at or near the distal end of the wand body. The electrosurgical conductor may pass through, between, or among any combination or singular component of the electrosurgical wand 300 including but not limited to the wand body 310, the lumen 320, the inner tubular member 322, the support coil 327, the passageway 325, and the transfer space 319. One or more electrosurgical conductors may implement monopolar or bipolar tissue ablation, tissue cauterizing, or any other effective electrosurgical procedure or technique. In embodiments where the support coil 327, or a similar support component, is a conductor between the controller 100 and a distal electrode or other electrically operable component of the electrosurgical wand 300, a wire of such a support coil may be insulated.

A side elevation view of an embodiment of the electrosurgical wand 300 of the electrosurgical system 1 with a distal portion of the electrosurgical wand 300 in a bent state is illustrated in FIGS. 7 and 8. FIG. 8 particularly illustrates a cross-sectional view of the bent distal portion of the electrosurgical wand 300. As evident in FIG. 8, a space 324 may develop between the outside diameter of the inner tubular member 322 and the inside diameter of the support coil 327 when the electrosurgical wand 300 is in a bent state. However, it is nonetheless valuable that embodiments of the present invention maintain a passageway 325 through the inner tubular member 322 when the electrosurgical wand 300 is in a bent state. In addition, while in a bent configuration, some portion of each revolution of the wire about the length of a support coil may no longer occur within a respective distance along the length of the support coil of three wire diameters. This may depend on how small or sharp the radius of curvature is formed. This is best seen in FIG. 8, showing support coil 327 extending along a bend, the bend defining an outer curve where the space 324 has opening up and inner curve. Not only may a space 324 open up, but also portions of the coil along this outer curve may spread to be further spaced from each other, while portions along the inner curve may be more tightly packed or relatively close along the inner radius. Therefore in the bent configuration, a portion of each revolution of the wire may occur within a respective distance along the length of the support coil of more than two wire diameters along the outer curve, and each revolution may spread to be up to four diameters, however still sufficient to shield the softer inner tubular member 322. Preferably, the support coil 327 may still have revolutions sufficiently close to prevent the inner tubular member from distending laterally outwards at the location of the bend. Additionally, in the bent configuration a portion of each revolution of the wire may occur within a respective distance along the length of the support coil of less than three wire diameters (inner curve).

In addition, a method of use may begin with placing a distal end of the wand near a target tissue, the wand distal end being oriented at a first angular orientation relative to a proximal end of the wand. This first orientation may be substantially straight or coaxial with the wand proximal end. Fluid may then be transferred along an inner tubular member of the wand, the inner tubular member having a support coil wrapped round the inner tubular member, as disclosed herein. The method may also include treating a target tissue electrosurgically and transferring treated tissue along the inner tubular member. The method further comprises plastically deforming a portion of the wand, such that the wand distal end is disposed at a second angular orientation relative to a proximal end of the wand, the wand configured to hold this second angular orientation. This second orientation is different from the first orientation. Fluid may then be transferred along the inner tubular member of the wand, the support coil wrapped round the inner tubular member as disclosed herein to support the inner tubular member and inhibit the inner tubular member from kinking. The method may further comprise plastically deforming a portion of the wand, such that the wand distal end is now oriented at a third angular orientation relative to a proximal end of the wand. This third orientation is different from at least one of the first or second orientation. Fluid may then be transferred along the inner tubular member of the wand, the support coil configured to inhibit the inner tubular member from kinking or deforming and thereby inhibit fluid flow. For example, the support coils assists in maintaining an opening along the inner tubular member to allow sufficient fluid to flow therethrough. In some embodiments, the steps of transferring fluid further comprises flowing treated tissue fragments therethough, and the support coil is configured to limit deformation of the inner tubular member to maintain a sufficient inner tubular member opening to allow passage of the tissue fragments therethrough.

Another embodiment of the disclosure is a method of manufacturing a medical device. The medical device may be, by way of non-limiting example, a lumen or an electrosurgical device that includes a lumen. Such method embodiments may include expanding a support coil, such as the support coil 327, with an inside diameter and an outside diameter along its length. The support coil 327 illustrated includes a helically formed wire with a wire diameter. The example wire illustrated extends helically along the length of the support coil 327 to produce walls of the support coil 327. The particular support coil 327 disclosed may be a standard medical grade spring such as an off-the-shelf extension spring. This type of spring is potentially much less expensive than a specially manufactured support coil, and even a greater savings compared to a support coil or other strand that has to be over-molded by another material to form the manufactured medical device.

The act of expanding the support coil 327 in some embodiments includes holding the support coil 327 at proximal and distal locations and applying a rotational force to the support coil 327 about an axis along the length of the support coil 327 in a direction opposite to the direction of winding of the helically formed wire that makes up the support coil 327. In other embodiments, expanding the support coil 327 may include applying a wedging force inside the support coil 327. For example, a wedged shaped mechanism such as a tapered mandrel may be forced inside the support coil 327 from one or both ends. In still other embodiments, expanding the support coil 327 may include placing an expandable mechanism in the support coil 327 and applying a force to increase the size of the expandable mechanism to a size that is large enough to expand the support coil 327.

The manufacturing method may also include inserting an inner tubular member, such as the inner tubular member 322 with an inside diameter and an outside diameter along its length into the support coil 327 inside diameter while the support coil 327 is expanded. Insertion of the inner tubular member 322 may occur while the support coil 327 is expanded to a size significantly larger than the outside diameter of the inner tubular member 322, or the fit may be tighter such that a lengthwise force is required to force the inner tubular member 322 into the support coil 327.

The manufacturing method may additionally include causing the support coil, such as the support coil 327, to constrict around the inner tubular member 322 such that the inside diameter of the support coil 327 closely conforms to the outside diameter of the inner tubular member 322 when the support coil 327 is substantially straight along substantially all of its length. An example straight support coil 327 conforming closely to the outside diameter of the inner tubular member 322 is shown in FIGS. 4-6. The act of causing the support coil 327, or other support coil embodiments, to constrict may include reducing a force used to expand the support coil 327. Such reductions of force may include one or more of reducing the counter-rotating force on the support coil 327, applying a twisting force to remove a wedge from one or more ends of the support coil 327, applying a tensile force to remove a wedge from one or more ends of the support coil 327, and any other effective force application or reduction.

In some embodiments, causing the support coil 327 to constrict includes waiting a period of time. For example, if the support coil 327 is made from a shape memory material, some period of time may need to be waited before an expanded support coil 327 will constrict around the inner tubular member 322. The period of time waited may also be merely the period of time after an inner tubular member 322 is moved into a support coil until the force used to move the components together is ended. Some acts causing the support coil 327 to constrict may also include changing the temperature of the support coil 327. In some embodiments, causing the support coil, such as support coil 327, to constrict includes applying a constricting force around the support coil 327. Causing the support coil 327 to constrict around the inner tubular member 322 may cause the distance between the inside diameter of the support coil 327 and the outside diameter of the inner tubular member 322 to be about 0.13mm on average. In other embodiments, causing the support coil 327 to constrict around the inner tubular member causes the support coil 327 and the inner tubular member 322 to be in contact along substantially the entire length of the support coil 327.

Other embodiments of the method may include coupling a lumen, such as the lumen 320, inside an electrosurgical wand, such as the electrosurgical wand 300, to provide a flexible and collapse-resistant passageway through the electrosurgical wand 300. The lumen 320 may be offered as a separate medical device or device component, or may be incorporated into the electrosurgical wand 300 and sold as part of the electrosurgical wand 300. One or both of the lumen 320 and the electrosurgical wand 300 may also be offered as part of the electrosurgical system 1.

Various embodiments of a system wholly or its components individually may be made from any biocompatible material. For example and without limitation, biocompatible materials may include in whole or in part: non-reinforced polymers, reinforced polymers, metals, ceramics, adhesives, reinforced adhesives, and combinations of these materials. Reinforcing of polymers may be accomplished with carbon, metal, or glass or any other effective material. Examples of biocompatible polymer materials include polyamide base resins, polyethylene, Ultra High Molecular Weight (UHMW) polyethylene, low density polyethylene, polymethylmethacrylate (PMMA), polyetheretherketone (PEEK), polyetherketoneketone (PEKK), a polymeric hydroxyethylmethacrylate (PHEMA), and polyurethane, any of which may be reinforced. Polymers used as bearing surfaces in particular may in whole or in part include one or more of cross-linked and highly cross-linked polyethylene. Example biocompatible metals include stainless steel and other steel alloys, cobalt chrome alloys, zirconium, oxidized zirconium, tantalum, titanium, titanium alloys, titanium-nickel alloys such as Nitinol and other superelastic or shape-memory metal alloys.

Terms such as proximal, distal, near, and the like have been used relatively herein. However, such terms are not limited to specific coordinate orientations, distances, or sizes, but are used to describe relative positions referencing particular embodiments. Such terms are not generally limiting to the scope of the claims made herein. Any embodiment or feature of any section, portion, or any other component shown or particularly described in relation to various embodiments of similar sections, portions, or components herein may be interchangeably applied to any other similar embodiment or feature shown or described herein.

While embodiments of the invention have been illustrated and described in detail in the disclosure, the disclosure is to be considered as illustrative and not restrictive in character.

## Claims

1. A surgical wand (300) comprising:
a wand body (310) with a proximal end (312) and a distal end (314), wherein the distal end (314) includes at least a portion that is flexible;
a fluid flow port (380) coupled near the proximal end (312) of the wand body (310) and communicating through the wand body (310) to facilitate the flow of fluid to or from the distal end (314) of the wand body (310);
a lumen (320) within at least a part of the portion of the distal end (314) that is flexible, the lumen (320) providing a passageway, the lumen (320) comprising:
an inner tubular member (322) in fluid communication with the fluid flow port (380) and with an inside diameter and an outside diameter along its length, and
a support coil (327) with an inside diameter and an outside diameter along its length, the support coil (327) comprising a helically formed wire with a wire diameter, the wire extending helically along the length of the support coil to produce walls of the support coil (327),
wherein the walls of the support coil (327) are defined by extents of the wire at the inside diameter of the support coil (327) and the outside diameter of the support coil (327), and
wherein substantially each revolution of the wire occurs within a respective distance along the length of the support coil (327) of three wire diameters;
wherein the flexible portion of the distal end (314) is plastically deformable and includes a malleable material that can retain a bent shape when positioned to that shape without continuing application of force while still providing support to the lumen (320).

2. The surgical wand of claim 1 wherein the fluid flow port (380) defines a suction port, for communicating through the inner tubular member (322) to facilitate the provision of negative pressure at or near the distal end (314) of the wand body (310).

3. The surgical wand of claim 1 wherein the material from which the inner tubular member (322) is made is more flexible than the material from which the support coil (327) is made.

4. The surgical wand of claim 1 wherein the inside diameter of the support coil (327) closely conforms to the outside diameter of the inner tubular member (322) when the support coil (327) is substantially straight along substantially all of its length.

5. The surgical wand of claim 1 wherein the support coil (327) and the inner tubular member (322) are in contact along substantially the entire length of the support coil (327).

6. The surgical wand of claim 5 wherein the support coil (327) must be radially expanded to fit around the inner tubular member (322).

7. The surgical wand of claim 1 wherein the wand further defines an electrosurgical wand, having an electrode disposed on the wand distal end (314).

8. An electrosurgical system comprising:
a controller (100); and
the surgical wand (300) of any of claims 1 to 7, wherein the surgical wand is electrically coupled to the controller (100) and fluidly coupled to a fluid control unit (200).

9. The electrosurgical system of claim 8 further comprising the fluid control unit (200), wherein the fluid control unit (200) is electrically coupled to the controller (100), the fluid control unit (200) configured to transfer fluids at a rate coordinated with operation of the controller (100).

## Patentansprüche

1. Ein chirurgischer Stab (300), der Folgendes umfasst:
einen Stabkörper (310) mit einem proximalen Ende (312) und einem distalen Ende (314), wobei das distale Ende (314) mindestens einen Anteil, der flexibel ist, einschließt;
eine Fluidstromöffnung (380), die in der Nähe des proximalen Ende (312) des Stabkörpers (310) gekoppelt ist und durch den Stabkörper (310) eine Verbindung herstellt, um den Fluidstrom zu dem oder von dem distalen Ende (314) des Stabkörpers (310) zu erleichtern;
ein Lumen (320) innerhalb mindestens eines Teils des Anteils des distalen Endes (314), das flexibel ist, wobei das Lumen (320) einen Durchgang bereitstellt, wobei das Lumen (320) Folgendes umfasst:
ein inneres röhrenförmiges Element (322) in Fluidverbindung mit der Fluidstromöffnung (380) und mit einem Innendurchmesser und einem Außendurchmesser entlang seiner Länge und
eine Stützspirale (327) mit einem Innendurchmesser und einem Außendurchmesser entlang ihrer Länge, wobei die Stützspirale (327) einen schraubenförmigen Draht mit einem Drahtdurchmesser umfasst, wobei sich der Draht schraubenförmig entlang der Länge der Stützspirale erstreckt, um Wände der Stützspirale (327) zu erzeugen,
wobei die Wände der Stützspirale (327) durch Ausdehnungen des Drahts an dem Innendurchmesser der Stützspirale (327) und dem Außendurchmesser der Stützspirale (327) definiert werden, und
wobei im Wesentlichen jede Umdrehung des Drahts innerhalb eines jeweiligen Abstands entlang der Länge der Stützspirale (327) von drei Drahtdurchmessern auftritt;
wobei der flexible Anteil des distalen Endes (314) plastisch verformbar ist und ein umformbares Material einschließt, das eine gebogene Gestalt, wenn sie in dieser Gestalt positioniert wird, ohne weitere Kraftanwendung aufrechterhalten kann, während sie weiterhin dem Lumen (320) eine Stütze bereitstellt.

2. Chirurgischer Stab nach Anspruch 1, wobei die Fluidstromöffnung (380) eine Absaugöffnung definiert, zum Herstellen einer Verbindung durch das innere röhrenförmige Element (322), um die Bereitstellung eines Unterdrucks an dem oder in der Nähe des distalen Endes (314) des Stabkörpers (310) zu erleichtern.

3. Chirurgischer Stab nach Anspruch 1, wobei das Material, aus dem das innere röhrenförmige Element (322) gefertigt ist, flexibler ist als das Material, aus dem die Stützspirale (327) gefertigt ist.

4. Chirurgischer Stab nach Anspruch 1, wobei der Innendurchmesser der Stützspirale (327) eng an den Außendurchmesser des inneren röhrenförmigen Elements (322) angepasst ist, wenn die Stützspirale (327) im Wesentlichen entlang ihrer gesamten Länge im Wesentlichen gerade ist.

5. Chirurgischer Stab nach Anspruch 1, wobei die Stützspirale (327) und das innere röhrenförmige Element (322) im Wesentlichen entlang der gesamten Länge der Stützspirale (327) in Kontakt sind.

6. Chirurgischer Stab nach Anspruch 5, wobei die Stützspirale (327) radial expandiert werden muss, damit sie um das innere röhrenförmige Element (322) herum passt.

7. Chirurgischer Stab nach Anspruch 1, wobei der Stab ferner einen elektrochirurgischen Stab definiert, der eine Elektrode aufweist, die auf dem distalen Ende (314) des Stabs angeordnet ist.

8. Ein elektrochirurgisches System, das Folgendes umfasst:
eine Steuereinheit (100); und
den chirurgischen Stab (300) nach einem der Ansprüche 1 bis 7, wobei der chirurgische Stab elektrisch mit der Steuereinheit (100) gekoppelt ist und fluidisch mit einer Fluidsteuereinheit (200) gekoppelt ist.

9. Elektrochirurgisches System nach Anspruch 8, das ferner die Fluidsteuereinheit (200) umfasst, wobei die Fluidsteuereinheit (200) elektrisch mit der Steuereinheit (100) gekoppelt ist, wobei die Fluidsteuereinheit (200) dazu ausgelegt ist, Fluide mit einer mit dem Betrieb der Steuereinheit (100) koordinierten Rate zu übertragen.

## Revendications

1. Baguette chirurgicale (300) comprenant :
un corps de baguette (310) ayant une extrémité proximale (312) et une extrémité distale (314), l'extrémité distale (314) comprenant au moins une portion qui est flexible ;
un orifice d'écoulement de fluide (380) couplé près de l'extrémité proximale (312) du corps de baguette (310) et communiquant à travers le corps de baguette (310) pour faciliter l'écoulement du fluide vers ou depuis l'extrémité distale (314) du corps de baguette (310) ;
une lumière (320) à l'intérieur d'au moins une partie de la portion de l'extrémité distale (314) qui est flexible, la lumière (320) formant un passage, la lumière (320) comprenant :
un élément tubulaire intérieur (322) en communication fluidique avec l'orifice d'écoulement de fluide (380) et ayant un diamètre intérieur et un diamètre extérieur le long de sa longueur, et
un enroulement de support (327) ayant un diamètre intérieur et un diamètre extérieur le long de sa longueur, l'enroulement de support (327) comprenant un fil formé de manière hélicoïdale ayant un diamètre de fil, le fil s'étendant de manière hélicoïdale le long de la longueur de l'enroulement de support pour former des parois de l'enroulement de support (327),
dans laquelle les parois de l'enroulement de support (327) sont délimitées par des étendues du fil au niveau du diamètre intérieur de l'enroulement de support (327) et du diamètre extérieur de l'enroulement de support (327), et
dans laquelle sensiblement chaque tour du fil couvre une distance correspondant à trois diamètres de fil le long de la longueur de l'enroulement de support (327) ;
dans laquelle la portion flexible de l'extrémité distale (314) est capable de subir une déformation plastique et contient un matériau malléable qui peut conserver une forme courbe lorsqu'il positionné de manière à avoir cette forme sans qu'une force ne soit appliquée en continu tout en fournissant encore un support à la lumière (320).

2. Baguette chirurgicale selon la revendication 1, dans laquelle l'orifice d'écoulement de fluide (380) délimite un orifice d'aspiration, pour une communication à travers l'élément tubulaire interne (322) afin de faciliter l'apport d'une pression négative à l'extrémité distale (314) ou à proximité de l'extrémité distale du corps de baguette (310).

3. Baguette chirurgicale selon la revendication 1, dans laquelle le matériau dont se compose l'élément tubulaire intérieur (322) est plus flexible que le matériau dont se compose l'enroulement de support (327).

4. Baguette chirurgicale selon la revendication 1, dans laquelle le diamètre intérieur de l'enroulement de support (327) se conforme étroitement au diamètre extérieur de l'élément tubulaire intérieur (322) lorsque l'enroulement de support (327) est sensiblement rectiligne sur pratiquement toute sa longueur.

5. Baguette chirurgicale selon la revendication 1, dans laquelle l'enroulement de support (327) et l'élément tubulaire interne (322) sont en contact sur pratiquement toute la longueur de l'enroulement de support (327).

6. Baguette chirurgicale selon la revendication 5, dans laquelle l'enroulement de support (327) doit être déployé radialement pour s'adapter aux contours de l'élément tubulaire intérieur (322).

7. Baguette chirurgicale selon la revendication 1, la baguette formant en outre une baguette électrochirurgicale, comportant une électrode disposée sur l'extrémité distale de la baguette (314).

8. Système électrochirurgical comprenant :
un dispositif de commande (100) ; et
la baguette chirurgicale (300) selon l'une quelconque des revendications 1 à 7, la baguette chirurgicale étant couplée électriquement au dispositif de commande (100) et couplée de manière fluidique à une unité de régulation de fluides (200).

9. Système électrochirurgical selon la revendication 8, comprenant en outre l'unité de régulation de fluides (200), l'unité de régulation de fluides (200) étant couplée électriquement au dispositif de commande (100), l'unité de régulation de fluides (200) étant configurée pour transférer des fluides à un débit coordonné avec le fonctionnement du dispositif de commande (100).
